# EUROPEAN PATENT SPECIFICATION

(11) **EP 1 194 163 B1**
(45) Date of publication and mention of the grant of the patent: **04.06.2014**
(21) Application number: 00945876.1
(22) Date of filing: 05.07.2000
(51) Int. Cl.: A61K 38/44, A61K 38/40, A61K 38/47, A61K 39/395, A61K 38/18, A61P 31/00

(54) **NOVEL METHODS AND MEDICAMENT FOR TREATING INFECTIOUS DISEASES INVOLVING MICROBIAL BIOFILMS**
NEUE METHODEN UND MEDIKAMENT ZUR BEHANDLUNG VON INFEKTIONSKRANKHEITEN BEDINGT DURCH MIKROBIELLE BIOFILME
NOUVEAUX PROCEDES ET MEDICAMENT POUR LE TRAITEMENT DE MALADIES INFECTIEUSES FAISANT INTERVENIR DES BIOFILMS MICROBIENS

(30) Priority: 07.07.1999 EP 99870145
(43) Date of publication of application: 10.04.2002
(73) Proprietor: Perraudin, Jean-Paul, 1180 Brussel (BE); Armor Proteines SAS, 50890 Condé-sur-Vire (FR)
(72) Inventor: Perraudin Jean-Paul, 1180 Brussel (BE)
(74) Representative: Pronovem
(86) International application number: PCT/EP2000/006312
(87) International publication number: WO 2001/003727

(56) References cited:
- WO-A-91/02533
- WO-A-96/34614
- WO-A-99/50391

## Description

### Field of the invention

The present invention relates to the improvement of prophylactic and therapeutic applications of innate and non-innate defence mechanisms (peroxidases, lactoferrin, lactoferrin peptides, lysozyme, immunoglobulins, alone or combined) in the presence of growth factor proteins (platelet derived growth factor, fibroblast growth factor, transforming growth factor, epidermal growth factor, angiogenin, alone or combined) for the control or treatment of micro-organisms, organised in biofilms, adherent to the cell surfaces.

### Background of the invention

The development of effective prophylactic and therapeutic agents for controlling micro-organism biofilms adherent to cell surfaces has proven problematic.

Prophylactic and therapeutic formulations and methods developed for the prevention of infections by control of the ecological microbial balance, in general, have only been partially successful.

It is well known that natural antimicrobial agents are contained in most natural external mammalian secretions. In particular, the naturally occurring antimicrobial thiocyanate/peroxidase/H₂O₂ systems, lactoferrin, lactoferrin peptides, lysozyme and immunoglobulins present in secretion liquids have been extensively studied.

Antimicrobial thiocyanate/peroxydase/ H₂O₂ systems produce hypothiocyanite (OSCN). These systems imitate the effect of the peroxidases (sialoperoxidase and myeloperoxidase) which catalyse the transformation of halide or pseudo-halide (as thiocyanate) into hypohalide or hypothiocyanite in the presence of the hydrogen peroxide produced by some bacterial strains.

Peroxidases and lactoperoxidase are known to stick to any support. There exists a paradox conflict between the *in vitro* cell toxicity and the *in vivo* clinical inefficiency data to decrease the bacterial count. Nevertheless, bacterial ATP content was shown in different species to decrease in vitro after halide and/or thiocynate/peroxidase/ H₂O₂ systems. This fact was confirmed in the human oral cavity after placement of a stick pill containing glucose/glucose-oxidase/thiocyanate/lactoperoxidase.

The resistance of oral mucosa to hypothiocyanite could be due to the protective role of biofilms on their surfaces. Some bacterial colonisers of these biofilms possess an NADH-hypothiocyanite-oxidoreductase (NHOR) activity, which can reduce hypothiocyanite. These bacteria with other. strains produce hydrogen peroxide.

So biofilms containing H₂O₂ producers, can stick peroxidase and NHOR activity acts as a protective screen avoiding colonisation by pathogenic micro-organisms and preserving tissue integrity. In many other cases, in the presence of several layers of biofilms, the upper layer protects the lower layer against the action of the antibacterial agents. This lower layer containing H₂O₂ producers will thus be responsible for the damage of the tissue composed of epithelial and fibroblast cells.

In nutrient-limited ecosystems, such as the aquatic environment, bacteria have a marked tendency to attach to surfaces and initiate the formation of a biofilm. These biofilms are also a severe problem in medical science, such as in oral health where they can cause dental plaque and periodontitis.

The biofilm is a collection of microcolonies with water channels in between and an assortment of cells and extra cellular polymers (glycoproteins, polysaccharides and proteins). The ability of the antimicrobial agents contained in saliva to react significantly on the bacteria organised in biofilms depends largely on the thickness of the biofilm.

In fact some of these agents are able to bind the bacteria so avoiding the adhesion process on the mucosal cells. However, these antimicrobial agents are not able to remove the biofilm.

Biofilms predominate in the oral environment and it is troubling that the biofilm phenotype of some species has been shown to differ radically from the planktonic phenotype of the same organism. One of the facets in which biofilm bacteria differ the most profoundly from their planktonic counterparts, is in the critical matter of resistance to antibacterial agents.

Results of in vitro studies showed Staphylococcus epidermis and Staphylococcus aureus were significantly more sensitive to the Lactoperoxidase system where the microorganisms are under planktonic cells than the biofilm cells, since the number of viable planktonic cells decrease by approximately 6 log units compared to a reduction of 1 log units or less in the number of biofilm cells.

The test results on the total bacteria count confirm that biofilm cells are more resistant than planktonic cells. This is believed to be due to a physical protection by the biofilm matrix or by an altered physiology of bacterial mode of growth.

In some experiments, bacterial activity either by Glucose Oxidase or Lactoperoxidase alone was observed. However, the daily variations in susceptibility of the biofilm cells may be explained by differences in the catalase activity and oxygen concentration.

In many cases, the bottom layer of biofilm will consist of anaerobic bacteria. As a result these biofilm cells may escape the inhibitory effect of the lactoperoxidase system, even though, under aerobic conditions, these cells have limited resistance to the lactoperoxidase system.

Moreover, the diffusion of thiocyanate and hydrogen peroxide into the biofilm will decrease the susceptibility of biofilm cells compared to planktonic cells. This suggests that the underlying cells of the biofilm will escape the antibacterial activity of the lactoperoxidase system unless the biofilms are released from the mucosal surface.

Such evidence may explain the difference in susceptibility between the bacteria in biofilm and the planktonic cells.

Lactoferrin is bacteriostatic by fixing ferric iron and making it unavailable for bacteria metabolism. Moreover lactoferrin presents a direct bactericidal effect on some micro-organisms but as long as that the microorganisms are organised in biofilm and that the biofilm can be protected by other biofilm layers, the lactoferrin has no or not sufficient antibacterial effect against the lower layer.

It is the main object of the present invention to provide an effective composition against biofilms, i.e. which compositions are able to desinfect surfaces.

Lysozyme hydrolyses proteoglycans in the bacterial cell walls causing cell lysis. Lysozyme has a synergistic effect in combination with lactoferrin aggregating cell suspensions of some bacterial strains. As long as the micro-organisms are organised in biofilms and are hidden by several layers of biofilms, lysozyme alone or in combination with lactoferrin has no effect against these micro-organisms.

Immunoglobulins are able to react specifically against the micro-organisms individually. The presence of several layers of biofilms and the characteristics of the biofilms avoid the action of the immunoglobulins alone or in combination with other antimicrobial agents such as lactoferrin to react against the individual micro-organisms. These different innate and non-innate antimicrobial molecules present a synergetic effect in vitro on bacteria suspension.

These different innate and non-innate antimicrobial molecules present a *synergetic* effect in vitro on bacteria suspension but not on bacteria organised in biofilm.

Secretion liquids have long been known to be active against a number of bacteria, viruses, yeast and protozoa. But, saliva supplementation with thiocyanate/peroxidase/H₂O₂ systems has been shown to be ineffective in vivo on the salivary bacterial count. It could be stressed that methodology errors are at the basis of these contradictory data; the biological effects of antimicrobial agents were tested on planctonic bacteria suspended in saliva but not on bacteria organised in biofilms.

No formulation has been successful for the control of biofilms, limiting so the occurrence and progression of infectious diseases. Only antibiotics and other drugs inhibiting bioadhesion have been investigated.

Thus, it can be seen that there remains a need for prophylactic and therapeutic agents to be associated in synergy with molecules to improve the activity of the epithelial and fibroblast cells able to remove bacterial biofilms, thus yielding a better accessibility to the antimicrobial molecule for control of the growth and pathogenic potential of the microorganisms.

Growth factors are well known in the art. Among these growth factors, the platelet derived growth factor (PDGF), a dimeric protein, is able to stimulate the growth of the fibroblast cells. The fibroblast growth factor (FGF), monomeric protein is able also to stimulate the growth of the fibroblast cells. The transforming growth factor (TGF), presented by different polypeptides are able to stimulate the growth of the fibroblast cells and the epithelial cells.

The epidermal growth factor (EGF) polypeptide is able to stimulate the growth of the epithelial cells.

Angiogenic factors are able to stimulate the growth of endothelial cells.

### Summary of the invention

It was surprisingly shown by the present inventors that in association or alone, different growth factors are able to stimulate the growth of the fibroblast and/or epithelial cells and so to reactivate the activity of these cells destroyed by the binding of the pathogenic micro-organisms organised in biofilms. This reactivation of the activity of fibloblast cells allows also the activation the epithelial cells which can be able by this action, to remove the micro-organism biofilms from their cell surfaces. This removing of the micro-organism biofilms yields them more accessible to the antibacterial, antiviral and candicidal actions of the peroxidase/H₂O_{2/}Halide or pseudohalide, lactoferrin, lactoferrin peptides, lysozyme and immunoglobulins alone or combined.

It is a primary object of the present invention to provide uses (applications) for peroxidases, lactoferrin, lactoferrin peptides, lysozymes and immunoglobulins (used alone and/or combined) for the control of microbial biofilms after the action of growth factors (used alone and/or combined) on the epithelial and fibroblast cells to remove microorganism biofilms adherent on their cell surfaces.

It is another primary object of the present invention to provide uses for peroxidases, lactoferrin, lactoferrin peptides, lysozymes and immunoglobulins (used alone and/or combined) combined with growth factors (used alone and/or combined) in the preparation (or manufacture) of medicaments for the prophylaxis or therapy of disease caused by microorganism present in biofilms adherent on cell surfaces.

Lactoferrin peptides are peptides produced by the action of a protease or a combination of proteases on lactoferrin.

It is another object of the present invention to provide peroxidases, lactoferrin, lactoferrin peptides, lysozymes and immunoglobulins (used alone and/or combined) combined with growth factors preferably chosen from platelet derived growth factor, fibroblast growth factor, transforming growth factor, angiogenin and epidermal growth factor (used alone and/or combined) in the preparation (or manufacture) of medicaments for the prophylaxis or therapy of disease caused by microorganisms present in biofilms adherent on cell surfaces.

It is another object of the present invention to provide a peroxide combined with a growth factor preferably chosen from platelet derived growth factor, fibroblast growth factor, transforming growth factor, angiogenin and epidermal growth factor (used alone and/or combined) in the preparation (or manufacture) of medicaments for the prophylaxis or therapy of disease caused by microorganisms present in biofilms adherent on cell surfaces.

It is another object of the present invention to provide lactoferrin or lactoferrin peptides combined with a growth factor preferably chosen from platelet derived growth factor, fibroblast growth factor, transforming growth factor, angiogenin and epidermal growth factor (used alone and/or combined) in the preparation (or manufacture) of medicaments for the prophylaxis or therapy of disease caused by microorganisms present in biofilms adherent on cell surfaces.

It is another object of the present invention to provide lysozyme combined with a growth factor preferably chosen from platelet derived growth factor, fibroblast growth factor, transforming growth factor, angiogenin and epidermal growth factor (used alone and/or combined) in the preparation (or manufacture) of medicaments for the prophylaxis or therapy of disease caused by microorganisms present in biofilms adherent on cell surfaces.

It is another object of the present invention to provide an immunoglobulin combined with a growth factor preferably chosen from platelet derived growth factor, fibroblast growth factor, transforming growth factor, angiogenin and epidermal growth factor (used alone and/or combined) in the preparation (or manufacture) of medicaments for the prophylaxis or therapy of disease caused by microorganisms present in biofilms adherent on cell surfaces.

It is another object of the present invention to provide peroxidases, lactoferrin, lactoferrin peptides, lysozymes and immunoglobulins (used alone and/or combined) combined with platelet derived growth factor in the preparation (or manufacture) of medicaments for the prophylaxis or therapy of disease caused by microorganisms present in biofilms adherent on cell surfaces.

It is another object of the present invention to provide peroxidases, lactoferrin, lactoferrin peptides, lysozymes and immunoglobulins (used alone and/or combined) combined with fibroblast growth factor in the preparation (or manufacture) of medicaments for the prophylaxis or therapy of disease caused by microorganisms present in biofilms adherent on cell surfaces.

It is another object of the present invention to provide peroxidases, lactoferrin, lactoferrin peptides, lysozymes and immunoglobulins (used alone and/or combined) combined with transforming growth factor in the preparation (or manufacture) of medicaments for the prophylaxis or therapy of diseases caused by microorganisms present in biofilms adherent on cell surfaces.

It is another object of the present invention to provide peroxidases, lactoferrin, lactoferrin peptides, lysozymes and immunoglobulins (used alone and/or combined) combined with epidermal growth factor in the preparation (or manufacture) of medicaments for the prophylaxis or therapy of disease caused by microorganisms present in biofilms adherent on cell surfaces.

It is another object of the present invention to provide peroxidases, lactoferrin, lactoferrin peptides, lysozymes and immunoglobulins (used alone and/or combined) combined with angiogenin in the preparation (or manufacture) of medicaments for the prophylaxis or therapy of disease caused by microorganisms present in biofilms adherent on cell surfaces.

A further object of the present invention is to provide a composition comprising colostrum and/or whey colostrum for providing a source of the growth factors.

It is another primary object of the present invention to provide prophylactic and therapeutic methods for controlling, preventing or treating infections in caused by microorganisms present in biofilms adherent on cell surfaces, by the administration of prophylactic and therapeutic effective amounts of peroxidases, lactoferrin, lactoferrin peptides, lysozymes and immunoglobulins (used alone and/or combined) and of prophylactic and therapeutic effective amounts of growth factors (used alone and/or combined) as defined above to individuals in need thereof.

As used herein, the term "prophylactic" refers variously to medicaments, amounts or quantities, methods, uses and effects, etc., that prevent and/or aid in preventing infections caused by the presence of microorganisms organized in biofilms adhered to cell surfaces. As used herein, the term "therapeutic" refers variously to medicaments, amounts or quantities, methods, uses and effects, etc., that ameliorate infections caused by the presence of microorganisms organized in biofilms adhered to cell surfaces.

These and other objects of the invention will become apparent from the following specification.

### Detailed description of the invention

The formulations (or medicaments) of the present inventions include peroxidase, lactoferrin, lactoferrin peptides, lysozyme and immunoglobulins on the one side and include proteins binding, by a means of a specific receptor, the epithelial and/or fibroblast cells and promoting growth of said cells such as for instance platelet derived growth factor (PDGF), fibroblast growth factor (FGF), transforming growth factor (TGF), angiogenin and epidermal growth factor (EGF) on the other side.

Preferably, the peroxidase component of the present invention includes a peroxidase/oxidizable substrate/hydrogen peroxide donor system that exhibits antiviral, antibacterial and candidicidal properties. In these formulations, peroxidase catalyses oxidation of the substrates (a halogen or pseudo-halogen) by a peroxide to form negatively charged, monovalent oxidizing compounds.

The substrates of the formulations of the present invention are chosen from a group consisting of negatively charged halogens, and their derivatives, and negatively charged pseudo-halogens, and their derivatives. The term "halogens" refers to certain of those elements, in their negatively-charged monovalent states, that belong to Group VII of the Periodic Table of Elements and, as is well known to those skilled in the art, includes bromide, chloride and iodide. The term "pseudo-halogens" refers to certain negatively charged ions and ionic compounds that are monovalent.

The "pseudo-halogens" of the present invention include the thiocyanate salts, such as sodium thiocyanate, potassium thiocyanate, ammonium thiocyanate, ferric thiocyanate and mixtures thereof.

The peroxidases present in the medicaments of the present invention include plant (vegetable) peroxidases, such as horseradish peroxidase, and mammalian peroxidases, such as salivary peroxidases, lactoperoxidases, myeloperoxidases and eosinophil peroxidase. These peroxidases may be extracted (isolated) from natural material (for example, saliva, human and bovine milk) or produced by natural of chemical methods, all of which are well known to those skilled in the art. These peroxidases also include those peroxidases that are produced by recombinant DNA techniques, also well known in the art. Human and bovine lactoperoxidase may for instance be produced by micro-organisms (for example transformed Pichia or transgenic animals such as transgenic cows) carring a cDNA expressing said protein.

Examples of the preferred peroxidase/substrate, combinations to be used in medicaments according to the present invention are also well known in the art. Examples of combinations are for instance referred to in US patent No. 4,564,519 and US patent 4,576,817.

As utilized herein, the term "International Unit(s)" identifies that amount of the enzyme that will effect catalysis of 1 micromole of substrate per minute at pH 7 and 25°C. Enzymes are supplied in dry or liquid form with the label specifying the concentration in IU's on a per gram or per milliliter basis, as appropiate.

The peroxidases present in formulations destined for adminstration to young animals may also comprise metalic peroxide donors, such as magnesium peroxide and sodium carbamide peroxide such as described in European patent application published under No. 0 290 410 in name of Ewos Aktiebolag.

The substrates of these peroxidases and their derivatives can be extracted (isolated) from natural material (for example, saliva and human milk and vegetables) or produced by natural of chemical methods, all of which are well known to those skilled in the art.

Examples of the preferred peroxidase/substrate combinations to utilize in the medicament of the present invention are set forth below in Table IA:

| TABLE IA | |
|---|---|
| Peroxidase | Substrates |
| (1) Salivary peroxidase | Thiocyanate, iodide |
| (2) Lactoperoxidase | Thiocyanate, iodide |
| (3) Myeloperoxidase | Chloride, iodide, thiocyanate |
| (4) Horseradish peroxidase | Chloride, iodide |
| (5) Plant peroxidase | Chloride, iodide, bromide |

The reactions of representative enzyme systems from Table IA (in the presence of a peroxide -which for purposes of illustration herein, will be hydrogen peroxide- from the oxygen donor) to produce either a hypohalite or hypothiocyanite compound, are set forth in Table IB, as follows:

| TABLE IB |
|---|
| (1a) Salivary peroxidase catalyzes the interaction of thiocyanate and hydrogen peroxide to produce hypothiocyanite and water; |
| (1b) Salivary peroxidase catalyzes the interaction of iodide and hydrogen peroxide to produce hypothiocyanite and water; |
| (2a) Lactoperoxidase catalyzes the interaction of thiocyanate and hydrogen peroxide to produce hypothiocyanite and water; |
| (2b) Lactoperoxidase catalyzes the interaction of iodide and hydrogen peroxide to produce hypothiocyanite and water; |
| (3a) Myeloperoxidase catalyzes the interaction of chloride and hydrogen peroxide to produce hypochlorite and water; |
| (3b) Myeloperoxidase catalyzes the interaction of iodide and hydrogen peroxide to produce hypoiodite and water; |
| (3c) Myeloperoxidase catalyzes the interaction of thiocyanate and hydrogen peroxide to produce hypotiocyanite and water; |
| (4a) Horseradish peroxidase catalyzes the interaction of chloride and hydrogen peroxide to produce hypochlorite and water; |
| (4b) Horseradish peroxidase catalyzes the interaction of iodide and hydrogen peroxide to produce hypochlorite and water; |
| (5a) Plant peroxidase catalyzes the interaction of chloride and hydrogen peroxide to produce hypochlorite and water; |
| (5b) Plant peroxidase catalyzes the interaction of iodide and hydrogen peroxide to produce hypoiodite and water; and |
| (5c) Plant peroxidase catalyzes the interaction of bromide and hydrogen peroxide to produce hypobromite and water. |

The oxygen donor of the present invention provides (supplies) the peroxide (for example, hydrogen peroxide) in the medicament necessary for oxidation of the substrate.

Preferably, the oxygen donor is an enzymatic system including a substrate, an enzyme specific to such substrate and other necessary reactants, such as water and/or oxygen and/or hydrogen. Alternatively, microorganisms, such as the Streptococci and Lactobacilli that are commonly referred to as lactic acid bacteria may be utilized in the medicaments of the present invention to supply the peroxide (in the form of hydrogen peroxide). Specific examples of such lactic acid bacteria include Lactobaccillus casei and Streptococcus faecalis and Streptococcus mutans. Use of such microorganisms (microbes) is especially preferred in the medicaments formulated for use as a vaginal cream for topical application.

It is also contemplated herein that inorganic peroxides (such as sodium peroxide and magnesium peroxide) or organic peroxides (such as benzyl peroxide and urea peroxide) may be utilized. Also, chemicals that, upon reaction, produce hydrogen peroxide may be utilized. Indeed, even hydrogen peroxide itself may be utilized as the oxygen donor. The precise oxygen donor to be utilized will vary depending upon several factors, including the formulation into which the medicament is to be made for administration.

Most preferably, the oxygen donor is an enzymatic system including an oxidizable substrate, an oxidoreductase enzyme specific to such substrate and other necessary reactants, such as oxygen and/or water. Examples of such oxidizable substrates, and oxidoreductase enzymes specific therefor, include those enumerated in United States Patent No. 4,564,519 issued to Pellico et all. Such examples are set forth below in Table IIA:

| TABLE IIA | | |
|---|---|---|
| Oxidizable substrate | Oxidoreductase enzyme | Other reactants |
| (a) B-D-glucose | Glucose oxidase | Water, Oxygen |
| (b) D-galactose | Galactose oxidase | Oxygen |
| (c) Urate | Urate oxidase | Water, Oxygen |
| (d) Choline | Choline oxidase | Oxygen |
| (e) D-amino acids¹ | D-amino acid oxidase | Water, Oxygen |
| (f) D-glutamate | D-glutamate oxidase | Water, Oxygen |
| (g) Glycine | Glycine oxidase | Water, Oxygen |
| (h) Glycollate | Glycollate oxidase | Water, Oxygen |
| (i) L-sorbose | L-sorbose oxidase | |
| (j) Primary alcohol | Alcohol oxidase | |
| (k) Primary amine | Amine oxidase | |
| (l) NAD(P)H | NAD(P)H oxidase | |
| (m) Oxygen free radical | Superoxide dismutase | |
| ¹D-amino acids includes D isomers of proline, methionine, isoleucine, alanine, valine and phenylalanine. | | |

The reactions of representative enzyme systems from Table IIA to produce hydrogen peroxide are set forth in Table IIB.

| TABLE IIB |
|---|
| (a) Glucose oxidase catalyzes the interaction of Beta-D-glucose, water and oxygen to produce hydrogen peroxide and gluconic add; |
| (b) Galactose oxidase catalyzes the interaction of D-galactose and oxygen to produce hydrogen peroxide and D-galacto-hexo-dioldose; |
| (c) Urate oxidase catalyze the interaction of urate, water and oxygen to produce hydrogen peroxide, allantoin and carbon dioxide; |
| (d) Choline oxidase catalyzes the interaction of choline and oxygen to produce hydrogen peroxide and betaine aldehyde; |
| (e) D-amino add oxidase catalyzes the interaction of D-amino adds, such as the D-isomers of proline, methionine, isoleudne, alanine, valine and phenylalanine together with water and oxygen to produce hydrogen peroxide, ammonia and the corresponding alpha-keto acids; |
| (f) D-glutamate oxidase catalyzes the interaction of D-glutamate, water and oxygen to produce hydrogen peroxide, ammonia and 2-oxoglutarate; and |
| (g) Glycine oxidase catalyzes the interaction of gtydne, water and oxygen to produce hydrogen peroxide, ammonia and glyoxylic add. |

The characteristics of representative oxidoreductase enzymes identified in Table IIA, from specific sources, are recited in US Patent No. 4,564,519.

Most preferably, the peroxidase medicaments of the present invention include either lactoperoxidase or myeloperoxidase in combination with a thiocyanate (SCN-) substrate and of a glucose/glucose oxidase enzymatic system oxygen donor.

It is preferred that the above-mentioned peroxidase/substrate/peroxide systems be formulated into the prophylactic and therapeutic medicaments for "in vivo" use as a substantially self-contained system that may be applied or used substantially without depending upon the users naturally-occurring "in vivo" concentrations of substrate, oxygen donors, peroxidases or other ingredients.

It is noted that the effectiveness of the peroxidase medicaments of the present invention may be effected by the naturally-occuring environment in which the medicament is to be administered. For example, in the human mouth, the concentration of hydrogen peroxide varies as a direct function of biological production and salivary flow. When salivary flow is at a diminished level, either as a natural event or as an event arising out of certain types of medical treatment, the oral concentrations of various elements, such as potassium thiocyanate and peroxidase, will be correspondingly reduced. This, in turn, may be a limiting factor in the prophylactic or therapeutic effectiveness of the medicament when it is orally administered. Moreover, when the oral concentration of peroxidase is suppressed through diminished salivary flow, oral concentrations of hydrogen peroxide may increase to a threshold level, wherein the hydrogen peroxide can impede the effectiveness of peroxidase of the medicament.

Accordingly, it can be seen that the concentrations of the substrate, oxygen donor and peroxidase in the medicaments described above should be adjusted and controlled to harmonize hydrogen peroxide and peroxidase concentration, so as to limit the hydrogen peroxide concentrations to levels which do not impede with the activity of the peroxidase.

As utilized herein, the term millimole identifies that quantity in grams corresponding to the molecular weight of the medicament divided by one thousand.

When the oxygen donor is hydrogen peroxide itself, it is generally present in the medicament of the present invention in an amount from about 2 to about 300 millimoles per gram of per milliliter of medicament and, preferably, from about 3 to about 30 millimole per gram or per milliliter of medicament.

In the event the oxygen donor is an oxidizable substrate and an oxidoreductase enzyme specific to the substrate, then the oxidizable substrate is generally present in the peroxidase medicament in an amount from about 0.015 to about 0.6 millimole per gram or per milliliter of medicament and, preferably, from about 0.025 to about 0.1 millimole per gram or per milliliter of medicament while the oxidoreductase is generally present in the medicament in an amount from about 0.5 to about 500 IU's per gram or per milliliter of the medicament and, preferably, from about 1.0 to about 40 IU per gram or per milliliter of the medicament.

In the event the oxygen donor is an organic or inorganic peroxide, then such peroxide is generally present in the medicament in an amount from about 0.000006 to about 0.6 millimole per gram or per milliliter of medicament and, preferably, from about 0.00006 to about 0.6 millimole per gram or per milliliter of medicament.

The substrate is generally present in the medicament in an amount ranging from about 0.0000008 to about 0.01 millimole per gram of per milliliter of medicament and, preferably, from about 0.000008 to about 0.006 millimole per gram of per milliliter of medicament.

In the event that the substrate is a thiocyanate salt (a pseudo-halogen), then it is generally present in the medicament in an amount from about 0.0001 to about 0.01 millimole per gram or per milliliter of medicament and, preferably, from about 0.001 to about 0.006 millimole per gram or per milliliter of medicament. Care should be taken in formulating the medicament, so as to avoid the use of metal compounds which inhibit or impair the effectiveness of the enzymes.

In the event that the substrate is a halogen, then it is generally present in the medicament in an amount from about 0.0000008 to about 0.008 millimole per gram or milliliter per medicament and, preferably, from about 0.000008 to about 0.004 millimole per gram of per milliliter of medicament.

The peroxidase is generally present in the medicaments in an amount from about 0.01 to about 50 IU per gram of per milliliter of medicament and, preferably, in an amount from about 0.2 to about 4.0 IU per gram or per milliliter of medicament.

It is noted that, if desired, the peroxidase medicament may be formulated for "in vivo" use as a system that relies upon certain naturally-occurring "in vivo" concentrations of any one or combination of compounds of the system for obtaining the peroxidase-generated compound.

The antiviral prophylactic and therapeutic qualities of the peroxidase medicaments of the present invention may be dependent on the concentration of compounds that are produced by the formulation of the medicament of the present invention. The produced concentrations of these compounds may vary between 1 micro molar and 100 millimolar, with concentrations of between 5 micro molar and 1 millimolar being preferred. For achieving this, the concentrations of the oxygen donor and/or of the substrate is able to be varied over a large range.

The presence of water promotes the oxidation/reduction reactions of the peroxidase medicaments of this invention. It also is a reactant in certain reactions. Thus, preferably, the use of water in formulating the said medicaments should be at a relatively low concentration levels in order to impart maximum stability and shelf life thereto.

Where the products of the activated enzyme systems in the medicaments include a weak organic acid, it is advantageous to formulate the medicament with a buffering agent to neutralize the organic acid. A suitable buffering agent is sodium bicarbonate.

In this regard, it is preferred that the peroxidase medicaments of the present invention should be formulated, so as to have a pH that substantially approximates physiological pH. In particular, it is preferred that the medicaments of the present invention have a pH ranging from 4.5 tot 6.5, with a pH of from 6 to 6.5 being especially preferred.

In the formulations according to the present invention, lactoferrin, presented under different iron saturated forms from 0 % of iron (apo-lactoferrin) to 100 % of iron saturation (Iron saturated lactoferrin) can be provided from different sources including for instance bovine lactoferrin from bovine secretions liquids for example bovine milk, human lactoferrin from human secretion liquids for example human milk, cDNA human like-lactoferrin or bovine like-lactoferrin produced by micro-organisms for example Pichia or from transgenic animals for example transgenis cows, all known in the art. Preferred dosages ranges of lactoferrin are from 0.001 g to 10 g, preferably from 0.01 g to 0.1 g per kg bodyweight per day or per 100 ml of liquid, gel, paste or other formulation.

Lactoferrin peptides are peptides produced by the action of a protease or a combination of proteases on lactoferrin. The proteases can be pepsin, chymotrypsin or any of all other proteases (proteolytic enzymes) used alone or in combined to proteolyse lactoferrin from any source such as described hereabove.

Useful concentration can be from 10 µgr to 10 gr of lactoferrin peptides per litre but preferably 100 µgr to 100 mgr of lactoferrin peptides per litre per kg bodyweight per day or per 100 ml of liquid, gel, paste or other formulation.

In these formulations, lysozyme can be provided from different sources including for instance bovine lysozyme from bovine secretion liquids for example bovine milk, human lysozyme from human secretion liquids for example human milk, egg white lysozyme from egg white for example hen egg white, cDNA human like-lysozyme or bovine like-lysozyme produced by microorganisms for example Pichia or from transgenic animals for example transgenic cows. Preferred dosages ranges of lysozyme are from 0.001 g to 50 g, preferably from 0.01 g to 10 g, more preferably from 0.01 g to 0.1 g per kg bodyweight per day or per 100 ml of liquid, gel, paste or other formulation.

In these formulations, immunoglobulins can be provided from different sources including for instance bovine immunoglobulins from bovine secretions liquids for example blood, colostrum, milk and other derivates, human immunoglobulins from human secretion liquids for example blood, milk and other derivates, egg immunoglobulins from egg yolk. That undertakes also immunoglobulins produced from secretion liquids of immunized animals. Purified immunoglobulin preparations known in the art and commercially available may also be used. Preferred dosages ranges of immunoglobulins are from 0.001 g to 1000 g, preferably from 0.001 g to 100 g, more preferably from 0.01 g to 10 g, most preferably from 0.05 g to 1 g per kg bodyweight per day or per 100 ml of liquid, gel, paste or other formulations.

These use of different of these above-mentioned compounds toghether has a synergetic effect.

In these formulations, the growth factor component can be provided from any source known in the art. Preferred dosages ranges of growth factors are from 1 ppb to 100 mg, preferably from 0.001 mg to 100 mg, more preferably from 0.01 mg to 10 mg, most preferably from 0.1 mg to 1 g per kg bodyweight per day or per 100 ml of liquid, gel, paste or other formulations.

In these formulations, the platelet derived growth factor can be provided from human or pig platelets or from bovine and human secretions liquids for example colostrum, milk and other derivates, manufactured by recombinant DNA technique, chemically synthesized, or a mixture thereof, all known in the art.

In these formulations, the fibroblast growth factor can be provided from hypophyse, brain, hypothalamus, retin surrenal gland, and kidney. He can also provide from bovine and human secretions liquids for example colostrum, milk and other derivates, manufactured by recombinant DNA techniques, chemically synthesized, or a mixture thereof, all known in the art.

In these formulations, the transforming growth factor can be provided from pig blood platelet, from bovine and human secretions liquids for example colostrum, milk and other derivates, manufactured by recombinant DNA techniques, chemically synthesized, or a mixture thereof, all known in the art.

In these formulations, the epidermal growth factor can be provided from different tissues and biological liquids of mammalian species, from bovine and human secretions liquids for example colostrum, milk and other derivates, manufactured by recombinant DNA techniques, chemically synthesized, or a mixture thereof, all known in the art.

In these formulations, the angiogenin growth factor can be provided from different tissues and biological liquids of mammalian species, from bovine and human secretions liquids for example colostrum, milk and other derivates, manufactured by recombinant DNA techniques, chemically synthesized, or a mixture thereof, all known in the art.

These use of different of these above-mentioned growth factors toghether has a synergetic effect.

The different possible formulations according to the present invention may be prepared for prophylactic and/or therapeutic purposes, as desired and needed, for permitting the administration of prophylactic and/or therapeutic effective amounts of the individual components thereof to an individual in need thereof for preventing and/or treating infections.

The formulation according to the present invention may be used to prevent and/or treat infections in humans or animals.

The formulations according to the present invention can be used for the prophylaxis or therapy of infectious diseases caused by micro-organisms forming biofilms on various types of human cell surfaces, such as for instance skin, ocular mucosa, orthorhino-laryngic spheres, gastro-enterologic cells and cell surfaces of the urogenital system.

The formulations according to the present invention are for instance useful for treating dental plaque, periodontal diseases, ulcers, tourista, bacterial vaginitis, vaginosis, cystitis, chlamydia infections.

The medicaments according to the present invention may be administrated in any form known in the art, and are for instance in the form of a topical medicament, an oral dentrifice or an injectable composition, and preferentially in the form of a gel, a stick pill, a rinsing liquid or a toothpaste, tablets, soft gelatin capsules, lozenges, powder mixtures, etc.

The pharmaceutical compositions or medicaments according to the present invention, and for use in accordance to the present invention, may comprise, in addition to the afore-mentioned compounds, a pharmaceutically acceptable excipient, carrier, buffer, stabiliser or other materials well known to those skilled in the art. Such materials should be non-toxic and should not interfere with the efficacy of the active ingredients. The precise nature of the carrier or other material may depend on the route of administration. Those of relevant skill in the art are well able to prepare suitable solutions.

In a therapeutic context, i.e. where the biological effect of the formulations to an individual is beneficial, administration is preferably in a "therapeutically effective amount", this being sufficient to show benefit to the patient. Such benefit may be at least amelioration of one symptom. The actual amount administered, and rate and time-course of the administration, will depend on the aim of the adminstration, e.g. the biological effect sought in view of the nature and severity of the challenge and is the subject of routine optimisation. Prescriptions of treatment, for example decisions on dosage etc., is within the responsibility of general practitioners and other medical doctors.

The medicaments of the present invention will be better understood by reference to the following examples.

### Brief Description of the Drawings

Figure 1 represents the bacterial count expressed in percentage in function of the time of incubation upon using stick pill formulations 1,2 and 3 as detailed in Example V.
Figure 2 represents the ATP percentage in function of the time of incubation upon using stick pill formulations 1, 2 and 3 as detailed in Example V.
Figure 3 represents the ratio of ATP/bacterial count in function of the time of incubation upon using stick pill formulations 1, 2 and 3 as detailed in Example V.
Figure 4 represents the bacterial count (CFU) in function of the time after brushing with toothpaste formulations 1, 2 and 3 as detailed In Example VI.

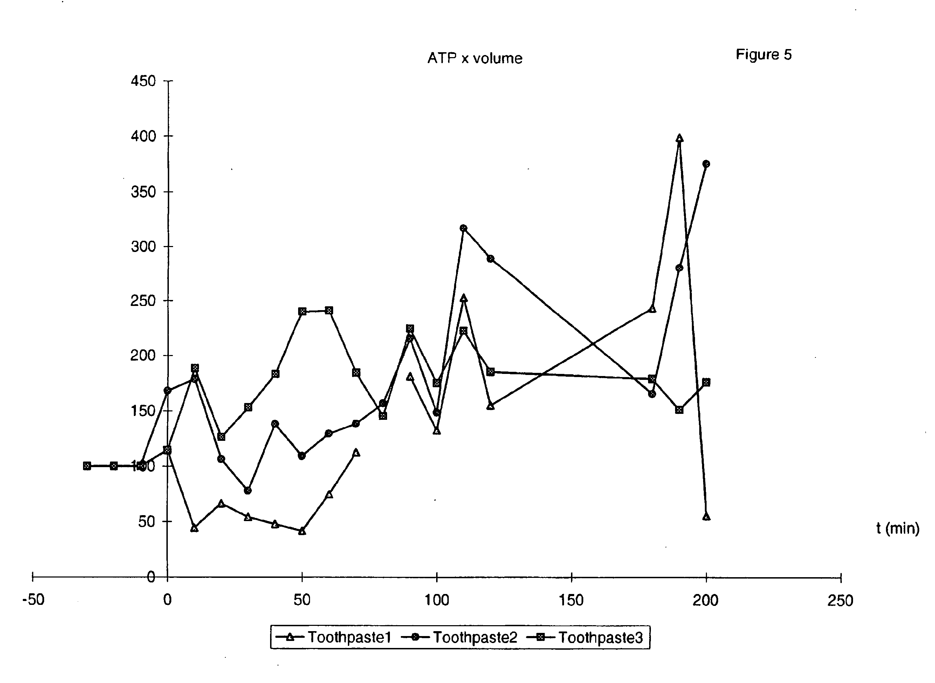

Figure 5 represents the ATP in function of the time after brushing with toothpaste formulations 1, 2 and 3 as detailed in Example VII.
Figure 6 represents the ATP/bacterial count (CFU) ratio in function of the time after brushing with the toothpaste formulations 1, 2 and 3 as detailed in Example VI.

### Examples

### Example 1

Illustrative base formulations for pharmaceutically-acceptable carriers for the peroxidase medicaments to be formulated with as a dentrifice for oral administration, such as a chewing gum and chewable tablets and lozenges are set forth in Table III, as follows:

| TABLE III | | | | |
|---|---|---|---|---|
| Ingredients | Weight, Percent | | | |
| | (a) | (b) | (c) | (d) |
| Sorbitol, crystalline | 75 | -- | 98 | 28 |
| Corn sugar | -- | 75 | -- | 70 |
| Gum base | 23 | 23 | -- | -- |
| Flavor | 1 | 1 | 1 | 1 |
| Color | 0.5 | 0.5 | 0.5 | 0.5 |
| Buffer | -- | -- | 0.5 | 0.5 |
| Saccharin, sodium | 0.005 | -- | 0.005 | -- |

In table III, formulations (a) and (b) illustrate pharmaceutically-acceptable carriers in the form of chewing gum compositions while formulations (c) and (d) illustrate pharmaceuticall-acceptable carriers in the form of tablet and lozenge compositions. Aspartame can be substituted for sodium saccharin in these formulations.

The following examples show varying ingredients and concentration levels which can be used in the preparation of dentrifices for providing the prophylactic and therapeutic effective amounts for oral administration according to the present invention:

**TABLE IV**

| Chewing gum: | Weight, Grams | | |
|---|---|---|---|
| Ingredients | 4A | 4B | 4C |
| Sorbitol | 70 | 70 | 70 |
| Gum base | 23 | 23 | 23 |
| Glycerol | 5 | 5 | 5 |
| Flavor | 1 | 1 | 1 |
| Color | 0.5 | 0.5 | 0.5 |
| Sodium Bicarbonate | 0.5 | 0.5 | 0.5 |
| | 100 | 100 | 100 |
| | | | |

| Enzymes: | | | |
|---|---|---|---|
| Glucose Oxidase | 40,000 IU | ----- | ----- |
| B-D Glucose | 1.0 g | ----- | ----- |
| Choline Oxidase | ----- | 8000 IU | ----- |
| Choline | ----- | 1.0 g | ----- |
| D-glutamate Oxidase | | | 2,500 IU |
| D-glutamate | | | 0.1 g |
| Lactoperoxidase | 4,000 IU | 1,500 IU | 1,000 IU |
| Potassium thiocyanate | 0.01 g | 0.005 g | |
| Sodium thiocyanate | | | 0.01 g |
| Lactoferrin | 0.1 g | 0.1 g | 0.1 g |
| Lysozyme | 0.1 g | 0.1 g | 0.1 g |
| Immunoglobulins | 1 g | 1 g | 1 g |
| | | | |

| Growth factors | | | |
|---|---|---|---|
| Derived Platelet growth factor | 0.01 mg | 0.01 mg | 0.01 mg |
| Transforming growth factor | 0.005 mg | 0.005 mg | 0.005 mg |
| Fibroblast growth factor | 0.01 mg | 0.01 mg | 0.01 mg |
| Epidermal growth factor | 0.015 mg | 0.015 mg | 0.015 mg |
| Angiogenin | 0.001 mg | 0.001 mg | 0.001 mg |

**TABLE V**

| Chewing gum: | Weight, Grams | | |
|---|---|---|---|
| Ingredients | 5A | 5B | 5C |
| Sorbitol, Cryst | 43 | 43 | 43 |
| Gum base | 20 | 20 | 20 |
| Glycerol | 25 | 25 | 25 |
| Flavor | 1 | 1 | 1 |
| Color | 0.5 | 0.5 | 0.5 |
| Sodium Bicarbonate | 0.5 | 0.5 | 0.5 |
| | 100 | 100 | 100 |
| | | | |

| Enzymes: | | | |
|---|---|---|---|
| D-amino acid oxidase | 5,000 IU | ---- | ---- |
| D-alanine | 0.1 g | ---- | ---- |
| Glucose oxidase | | 20,000 IU | 2,000 IU |
| B-D-Glucose | ---- | 0.5 g | 0.5 g |
| Lactoperoxidase | 4,000 IU | 1,500 IU | 1,000 IU |
| Potassium thiocyanate | 0.01 g | 0.005 g | |
| Sodium thiocyanate | | | 0.01 g |
| Lactoferrin | 0.1 g | 0.1 g | 0.1 g |
| Lysozyme | 0.1 g | 0.1 g | 0.1 g |
| Immunoglobulins | 1 g | 1 g | 1 g |
| | | | |

| Growth factors | | | |
|---|---|---|---|
| Derived Platelet growth factor | 0.01 mg | 0.015 mg | 0.005 mg |
| Transforming growth factor | 0.005 mg | 0.0025 mg | 0.01 mg |
| Fibroblast growth factor | 0.015 mg | 0.01 mg | 0.005 mg |
| Epidermal growth factor | 0.015 mg | 0.01 mg | 0.005 mg |
| Angiogenin | 0.001 mg | 0.001 mg | 0.001 mg |

**TABLE VI**

| Lozenges: | Weight, Grams | | |
|---|---|---|---|
| Ingredients | 6A | 6B | 6C |
| Sorbitol, Crystalline | 97 | 97 | 97 |
| Glycerol | 1 | 1 | 1 |
| Flavor | 1 | 1 | 1 |
| Color | 0.5 | 0.5 | 0.5 |
| Sodium Bicarbonate | 0.5 | 0.5 | 0.5 |
| | 100 | 100 | 100 |
| | | | |

| Enzymes : | | | |
|---|---|---|---|
| Glucose Oxidase | 10,000 IU | ----- | ----- |
| B-D Glucose | 1.0 g | ----- | ----- |
| Choline Oxidase | ----- | ----- | 2,000 IU |
| Choline | ----- | ----- | 0.5 g |
| Uranate oxidase | ----- | 10,000 IU | ----- |
| Urate | ----- | 0.75 g | ----- |
| Lactoperoxidase | 2,000 IU | 2,000 IU | 1,000 IU |
| Potassium thiocyanate | ----- | ----- | 0.01 g |
| Sodium thiocyanate | 0.01 g | 0.01 g | ----- |
| Lactoferrin | 0.1 g | 0.2 g | 0.05 g |
| Lysozyme | 0.1 g | 0.2 g | 0.05 g |
| Immunoglobulins | 1 g | 1 g | 1 g |
| | | | |
| | | | |

| Growth factors | | | |
|---|---|---|---|
| Derived Platelet growth factor | 0.01 mg | 0.01 mg | 0.01 mg |
| Transforming growth factor | 0.005 mg | 0.005 mg | 0.005 mg |
| Fibroblast growth factor | 0.01 mg | 0.01 mg | 0.01 mg |
| Epidermal growth factor | 0.015 mg | 0.015 mg | 0.015 mg |
| Angiogenin | 0.001 mg | 0.001 mg | 0.001 mg |

**TABLE VII**

| Lozenges: | Weight, Grams | | |
|---|---|---|---|
| Ingredients | 7A | 7B | 7C |
| Sorbitol, Crystalline | 80 | 80 | 80 |
| Corn Sugar | 17 | 17 | 17 |
| Flavor | 1 | 1 | 1 |
| Color | 0.5 | 0.5 | 0.5 |
| Sodium Bicarbonate | 0.5 | 0.5 | 0.5 |
| | 100 | 100 | 100 |
| | | | |

| Enzymes : | | | |
|---|---|---|---|
| D-glutamate oxidase | 10,000 IU | ----- | ----- |
| D-glutamate | 0.05 g | ----- | ----- |
| Glucose Oxidase | ----- | 5,000 IU | 1,000 IU |
| B-D Glucose | ----- | 0.5 g | 1 g |
| Lactoperoxidase | 1,500 IU | 2,000 IU | 1,000 IU |
| Potassium thiocyanate | 0.001 g | 0.005 g | ----- |
| Sodium thiocyanate | ----- | ----- | 0.005 g |
| Lactoferrin | 0.1 g | 0.1 g | 0.1 g |
| Lysozyme | 0.1 g | 0.1 g | 0.1 g |
| Immunoglobulins | 1 g | 1 g | 1 g |
| | | | |

| Growth factors | | | |
|---|---|---|---|
| Derived Platelet growth factor | 0.01 mg | 0.015 mg | 0.005 mg |
| Transforming growth factor | 0.005 mg | 0.0025 mg | 0.01 mg |
| Fibroblast growth factor | 0.015 mg | 0.01 mg | 0.005 mg |
| Epidermal growth factor | 0.015 mg | 0.01 mg | 0.005 mg |
| Angiogenin | 0.001 mg | 0.001 mg | 0.001 mg |

**TABLE VIII**

| Soft gelatin capsules: | Weight, Grams | | |
|---|---|---|---|
| Ingredients | 8A | 8B | 8C |
| Onager oil | 0.275 g | ----- | ----- |
| Vit C | 0.06 g | 0.06 g | 0.06 g |
| Vit E | 0.01 g | 0.01 g | 0.01 g |
| Beta carotene | 0.0001 g | 0.0001 g | 0.0001 g |
| Selenium | 0.0001 g | 0.0001 g | 0.0001 g |
| Soja oil | ----- | 0.275 g | ----- |
| Fish oil (23% DHA) | ----- | ----- | 0.275 g |
| | | | |

| Enzymes: per 800 mg soft gelatin capsules | | | |
|---|---|---|---|
| Superoxide dismutase | 100 IU | 500 IU | 1,000 IU |
| Lactoperoxidase | 1,500 IU | 2,000 IU | 1,000 IU |
| Potassium thiocyanate | 0.01 g | 0.05 g | ----- |
| Sodium thiocyanate | ----- | ----- | 0.05 g |
| Lactoferrin | 0.05 g | 0.1 g | 0.1 g |
| Lysozyme | 0.05 g | 0.1 g | 0.1 g |
| Immunoglobulins | 0.1 g | 0.05 g | 0.1 g |
| | | | |

| Growth factors | | | |
|---|---|---|---|
| Derived Platelet growth factor | 0.01 mg | 0.015 mg | 0.005 mg |
| Transforming growth factor | 0.005 mg | 0.0025 mg | 0.01 mg |
| Fibroblast growth factor | 0.015 mg | 0.01 mg | 0.005 mg |
| Epidermal growth factor | 0.015 mg | 0.01 mg | 0.005 mg |
| Angiogenin | 0.001 mg | 0.001 mg | 0.001 mg |

### Example II

Illustrative base formulations for pharmaceutically-acceptable carriers for the peroxidase medicaments of the present invention to be formulated as a topical medicament for topical administration, such as a cream, a gel or to be incorporated in a bandage or pad, are set forth in Table IX, as follows:

**TABLE IX**

| Gel | Weight, Percent | |
|---|---|---|
| | | |
| Ingredients | 9A | 9B |
| Deionized water | 19.02 | 20.0 |
| Corn Starch¹ | 38.04 | ----- |
| Lubrajel DV² | 38.04 | ----- |
| Aloe vera | 0.000021 | ----- |
| Natrosol 250 M³ | 0.1 | ----- |
| Xylitol | 4.76 | ----- |
| Cirami N.1⁴ | ----- | 20.0 |
| Sunflower Oil | ----- | 40.0 |
| Vitamin E | ----- | 0.05 |
| Tensami 4/07⁴ | ----- | 2.0 |
| Tensami 1/05⁴ | ----- | 3.0 |
| Bronopol⁴ | ----- | 2.0 |
| Myacide SP⁴ | ----- | 2.0 |
| Propylene Glycol | ----- | 10.0 |

| | | |
|---|---|---|
| ¹ An example of such a corn starch is the hydrogenated starch solution marketed under the name HYSTAR TPF by Alban Muller International, Montreuil, France. ² Lubragel DV is a Glycerine and acrylic solution marketed by Alban Muller International, Montreuil, France. ³ Natrosol 250 M is a hydroxeyethycellulose marketed by Aqualon, Inc., of Hopewell, Virginia, U.S.A. ⁴ Cirami N.1, Tensami 4/07, Tensami 1/05, Bronopol and Myacide SP are all marketed by Alban Muller International, Montreuil, France. | | |

In table IX, formulation (a) illustrates pharmaceutically-acceptable carriers in the form of a gel, and (b) illustrates pharmaceutically-acceptable carriers in the form of a cream.

The following tables show varying ingedients and prophylactic and therapeutic effective amounts (quantities) which can be used in the preparation of topical peroxidase medicaments, according to the present invention:

**TABLE X**

| Gel: | Weight, Grams | |
|---|---|---|
| Ingredients | 10A | 10B |
| Deionized water | 19.03 | 19.03 |
| Corn Starch | 38.054 | 38.054 |
| Lubrajel DV | 38.054 | 38.054 |
| Aloe vera | 0.001 | 0.001 |
| Natrosol 250 M | 0.1 | 0.1 |
| Xylitol | 4.76 | 4.76 |
| | 100 | 100 |

| Enzymes: | | |
|---|---|---|
| Glucose Oxidase | 10,000 IU | ----- |
| Glucose | 1.0 g | ----- |
| Choline Oxidase | ----- | 8,000 IU |
| Choline | ----- | 1.0 g |
| Lactoperoxidase | 2,000 IU | 1,500 IU |
| Potassium thiocyanate | ----- | 0.005 g |
| Sodium thiocyanate | 0.05 g | ----- |
| Lactoferrin | 0.05 g | 0.1 g |
| Lysozyme | 0.05 g | 0.1 g |
| Immunoglobulins | 0.1 g | 0.1 g |
| | | |

| Growth factors | | |
|---|---|---|
| Derived Platelet growth factor | 0.01 mg | 0.005 mg |
| Transforming growth factor | 0.005 mg | 0.01 mg |
| Fibroblast growth factor | 0.015 mg | 0.005 mg |
| Epidermal growth factor | 0.015 mg | 0.005 mg |
| Angiogenin | 0.001 mg | 0.001 mg |

**TABLE XI**

| Cream: | Weight, Grams | | |
|---|---|---|---|
| Ingredients | 11A | 11B | 11C |
| Deionized Water | 21.51 | 21.51 | 21.51 |
| Cirami N. | 20.0 | 20.0 | 20.0 |
| Sunflower Oil | 40.0 | 40.0 | 40.0 |
| Vitamin E | 0.04 | 0.04 | 0.04 |
| Tensami 4/07 | 2.0 | 2.0 | 2.0 |
| Tensami 1/05 | 3.0 | 3.0 | 3.0 |
| Bronopol | 2.0 | 2.0 | 2.0 |
| Myacide SP | 2.0 | 2.0 | 2.0 |
| Propylene Glycol | 10.0 | 10.0 | 10.0 |
| | 100 | 100 | 100 |
| | | | |

| Enzymes: | | | |
|---|---|---|---|
| Glucose Oxidase | 5.000 IU | ----- | ----- |
| Glucose | 0.5 g | ----- | ----- |
| | | | |
| D-amino acid oxidase | ----- | 5,000 IU | ----- |
| D-alanine | ----- | 0.1 g | ----- |
| Urate oxidase | ----- | ----- | 10,000 IU |
| Urate | ----- | ----- | 0.75 g |
| Lactoperoxidase | 2,000 IU | 1,000 IU | 1,500 IU |
| Potassium thiocyanate | 0.005 g | ----- | ----- |
| Sodium thiocyanate | ----- | 0.01 g | 0.08 g |
| Lactoferrin | 0.05 g | 0.1 g | 0.1 g |
| Lysozyme | 0.05 g | 0.1 g | 0.1 g |
| Immunoglobulins | 0.1 g | 0.05 g | 0.1 g |
| | | | |

| Growth factors | | | |
|---|---|---|---|
| Derived Platelet growth factor | 0.01 mg | 0.015 mg | 0.005 mg |
| Transforming growth factor | 0.005 mg | 0.0025 mg | 0.01 mg |
| Fibroblast growth factor | 0.015 mg | 0.01 mg | 0.005 mg |
| Epidermal growth factor | 0.015 mg | 0.01 mg | 0.005 mg |
| Angiogenin | 0.001 mg | 0.001 mg | 0.001 mg |

**TABLE XII**

| Cream: | Weight, Grams | | |
|---|---|---|---|
| Ingredients | 12A | 12B | 12C |
| Deionized Water | 21.51 | 21.51 | 21.51 |
| Cirami N. | 20.0 | 20.0 | 20.0 |
| Sunflower Oil | 40.0 | 40.0 | 40.0 |
| Vitamin E | 0.04 | 0.04 | 0.04 |
| Tensami 4/07 | 2.0 | 2.0 | 2.0 |
| Tensami 1/05 | 3.0 | 3.0 | 3.0 |
| Bronopol | 2.0 | 2.0 | 2.0 |
| Myacide SP | 2.0 | 2.0 | 2.0 |
| Propylene Glycol | 10.0 | 10.0 | 10.0 |
| | 100 | 100 | 100 |
| | | | |

| Enzymes: | | | |
|---|---|---|---|
| Superoxide dismutase | 500 IU | 1,000 IU | 2,000 IU |
| Lactoperoxidase | 2,000 IU | 1,000 IU | 1,500 IU |
| Potassium thiocyanate | 0.005 g | ----- | ----- |
| Sodium thiocyanate | ----- | 0.01 g | 0.08 g |
| Lactoferrin | 0.05 g | 0.1 g | 0.1 g |
| Lysozyme | 0.05 g | 0.1 g | 0.1 g |
| Immunoglobulins | 0.1 g | 0.05 g | 0.1 g |

### Example III

Illustrative formulations for pharmaceutically-acceptable carriers for the peroxidase medicaments of the present invention to be formulated as an eye wash solution for topical administration, as an eye drop or as an eye wash, are set forth in Table XIII, as follows:

The following Table shows the varying ingredients and the prophylactic and therapeutic effective amounts (quantities) which can be used in the preparation of eye wash medicaments, according to the present invention:

**TABLE XIII**

| Eye drop or eye wash (per 5 ml solution) | | |
|---|---|---|
| | Weight, Percent | |
| Ingredients | 13A | 13B |
| Sorbic Acid 0.0025% | ----- | 0.0002 |
| Pudfied Water | 99.4 | 98.1 |
| Boric Acid | 0.018 | 0.0176 |
| Sodium Borate (Hydrated 10 H₂O) | 0.0015 | 0.0013 |
| Sodium Chloride | 0.0025 | **-----** |
| Benzalkonium chloride¹ | 0.0001 | ----- |
| Edetate disodium¹ | 0.001 | **-----** |

| | | |
|---|---|---|
| ¹ Benzalkonium chloride and Edetate disodium are added as preservatives. | | |

| Enzymes: (amounts per 5 ml Eye Wash¹) | | |
|---|---|---|
| Glucose oxidase | 2,500 IU² | |
| Glucose | 0.02 g | |
| Superoxide dismutase | | 100 IU |
| Lactoperoxidase | 200,000 ABTS units³ | 150,000 ABTS units³ |
| Potassium thiocyanate | | 0.0005 g |
| Sodium thiocyanate | 0.0005 g | |
| Lactoferrin | 0.0001 g | 0.00005 g |
| Lysozyme | 0.0001 g | 0.00005 g |
| Immunoglobulins | 0.0001 g | 0.00005 g |
| | | |

| Growth factors | | |
|---|---|---|
| Derived Platelet growth factor | 0.001 mg | 0.0005 mg |
| Transforming growth factor | 0.0005 mg | 0.0001 mg |
| Fibroblast growth factor | 0.0015 mg | 0.0005 mg |
| Epidermal growth factor | 0.0015 mg | 0.0005 mg |
| Angiogenin | 0.0001 mg | 0.0001 mg |

| | | |
|---|---|---|
| ¹ The eye wash solution is a 5 ml solution of: 90 milligrams of Boric acid; 6.6 milligrams of hydrated sodium borate (10 H₂O); 2500 units Vitamin A and 0.125 µg of sorbic acid 0.0025% ² As utilized in this example, the term "unit" of Glucose oxidase identifies that amount of Glucose oxidase that oxidizes 3.0 milligram glucose to gluconic acid in one minute at pH 5.10 and 37°C. The assay conditions are set forth in Assay method FS 250 of Finnish Sugar Co. Ltd., of Finland. In this Example, 1 milligram of glucose oxidase has an activity of 100-120 units at 37°C at pH5. ³ As utilized herein, the term "ABTS units" identifies that amount of lactoperoxidase that catalyzes the oxidation of 1 mM of the ABTS substrate (2,2' -Azino-bis (3-Ethylbenzthiazoline-6-Sulfonic Acid)) in one minute at pH 5 and 37°C. The assay conditions are set forth by Mansson-Rahemtulla, B., et al., Biochemistry, Vol. 27, at pages 233-239 (1988). In this Example, 1 milligram of lactoperoxidase has an activity of 600 ABTS units at 37°C and 5 pH. | | |

In case of the formulation 13A, the composition is formulated separately in two parts which, before application, are combined and shaken to dissolve and mix the two parts.

The first part is a mixture of the lactoperoxidase and the glucose oxidase. The second part is a 5 ml solution of the boric acid, hydrated sodium borate (10 H₂O) Vitamin A, 0.0025% sorbic acid, potassium thiocyanate, water and glucose. The 5 ml solution (the second part) is mixed with the first part and shaken to dissolve the powder. Administration may be made as normal eye drops.

### Example IV

Illustrative formulations for pharmaceutically-acceptable carriers for the peroxidase medicaments of the present invention to be formulated as a tooth paste for pets and a feed complement for calves, are set forth in Table XIV and XV, as follows:

**TABLE XIV**

| Toothpase for pets (per 100 g of paste) | Weight, Grams | |
|---|---|---|
| Ingredients: | 14A | 14B |
| Malt | 10 g | 10 g |
| Sorbitol | 40 g | 40 g |
| Glycerin | 10 g | 10 g |
| Water | 2 g | 2 g |
| Xanthan Gum | 6 g | 6 g |
| Calcium di phosphate | 10 g | 10 g |
| Silica | 10 g | 10 g |
| Emusifier | 12 g | 12 g |
| | | |

| Enzymes: | | |
|---|---|---|
| Glucose oxidase | 10,000 IU | 2,000 IU |
| Glucose | 0.2 g | 0.25 g |
| Lactoperoxidase | 10,000 ABTS units (3) | 15,000 ABTS units (3) |
| Potassium thiocyanate | | 0.0005 g |
| Sodium thiocyanate | 0.0015 g | |
| Lactoferrin | 0.01 g | 0.005 g |
| Lysozyme | 0.01 g | 0.005 g |
| Immunoglobulins | 0.1 g | 0.05 g |
| | | |

| Growth factors | | |
|---|---|---|
| Derived Platelet growth factor | 0.001 mg | 0.0005 mg |
| Transforming growth factor | 0.0005 mg | 0.0001 mg |
| Fibroblast growth factor | 0.0015 mg | 0.0005 mg |
| Epidermal growth factor | 0.0015 mg | 0.0005 mg |
| Angiogenin | 0.0001 mg | 0.0001 mg |

**TABLE XV**

| Feed complements for calves | | |
|---|---|---|
| (per 100 g of powder) | Weight, Grams | |
| Ingredients: | 15A | 15B |
| Milk powder | 42.5 g | 42.5 g |
| Bovine colostrum | 42.5 g | 42.5 g |
| | | |

| Enzymes: | | |
|---|---|---|
| Glucose oxidase | 12,000 IU (2) | 10,000 IU (2) |
| Glucose | 0.2 g | 0.25 g |
| Lactoperoxidase | 10,000 ABTS units (3) | 15,000 ABTS units (3) |
| Potassium thiocyanate | | 0.0005 g |
| Sodium thiocyanate | 0.0015 g | |
| Lactoferrin | 0.01 g | 0.005 g |
| Lysozyme | 0.01 g | 0.005 g |
| Immunoglobulins | 12 g | 15 g |
| | | |

| Growth factors | | |
|---|---|---|
| Derived Platelet growth factor | 0.001 mg | 0.0005 mg |
| Transforming growth factor | 0.0005 mg | 0.0001 mg |
| Fibroblast growth factor | 0.0015 mg | 0.0005 mg |
| Epidermal growth factor | 0.0015 mg | 0.0005 mg |
| Angiogenin | 0.0001 mg | 0.0001 mg |

### Example V

This example shows the effectiveness of the enzyme group (Peroxidase/substrate/peroxide system - Lactoferrin - Lysozyme - Immunoglobulins) in combination with the growth factors (Platelet Derived growth factor - Fibroblast growth factor - Transforming growth factor - Epidermal growth factor - Angiogenin) of the present invention against oral micro-organisms.

### Stick Pills with active ingredients Enzymes and Growth Factors : Stick 1

| Active Ingredients | Amounts per stick pill |
|---|---|
| Lactoperoxidase | 100 IU |
| Glucose Oxidase | 26 IU |
| B-D-Glucose | 25 mg |
| Substrate (KI) | 1,1 mg |
| Lactoferrin | 10 mg |
| Lysozyme | 10 mg |
| Immunoglobulins | 30 mg |
| Platelet Derived growth factor | 10 ng |
| Transforming growth factor | 12 ng |
| Fibroblast growth factor | 5 ng |
| Epidermal growth factor | 100 ng |
| Anglogenin | 0.1 ng |

| | |
|---|---|
| Non active ingredients Carbopol^{®} 974P, Drum-Dried-Waxy-Maire Starch | |

Stick Pills with only the Enzymes as active ingredients : Stick 2

| Active Ingredients | Amounts per stick pill |
|---|---|
| Lactoperoxidase | 100 IU |
| Glucose Oxidase | 26 IU |
| Glucose | 25 mg |
| Substrate (KI) | 1,1 mg |
| Lactoferrin | 10 mg |
| Lysozyme | 10 mg |
| Immunoglobulins | 30 mg |

### Non active ingredients

### Carbopol 974P, Drum-Dried-Waxy-Maire Starch

Stick Pills where the active ingredients is replaced by maltodextrin : Stick 3 (controle)

### Non active ingredients

### Carbopol 974P, Drum-Dried-Waxy-Maire Starch

The Stick Pills 1 to 3 were applied to patients suffering from a dry mouth because they produced less than 0,2 ml per minute of saliva. The Stick Pill is sticked to the gum and allows a slow release of the active ingredients during several hours in the mouth of the patients. 24 patients were used in each group.

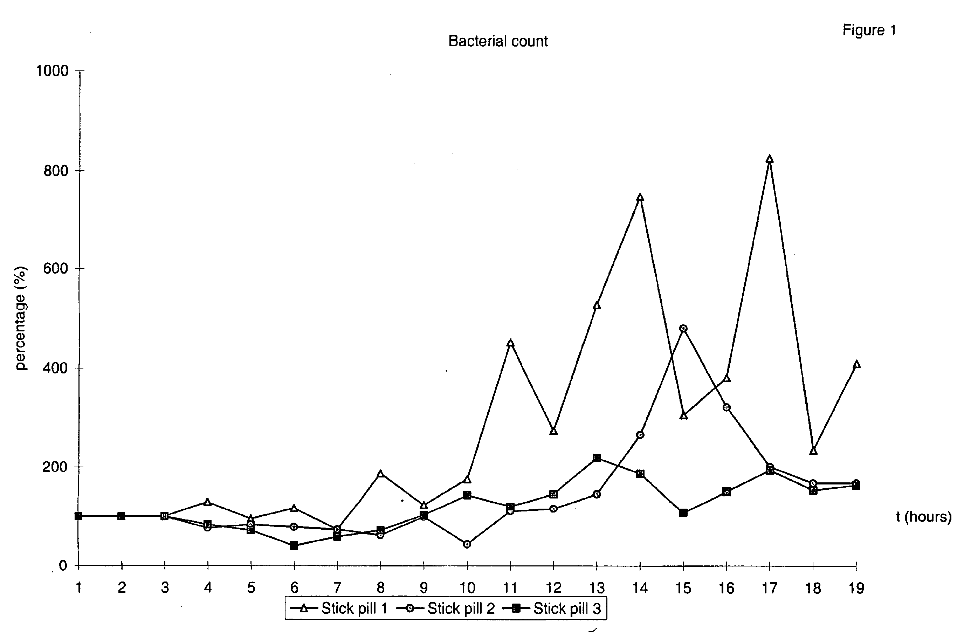

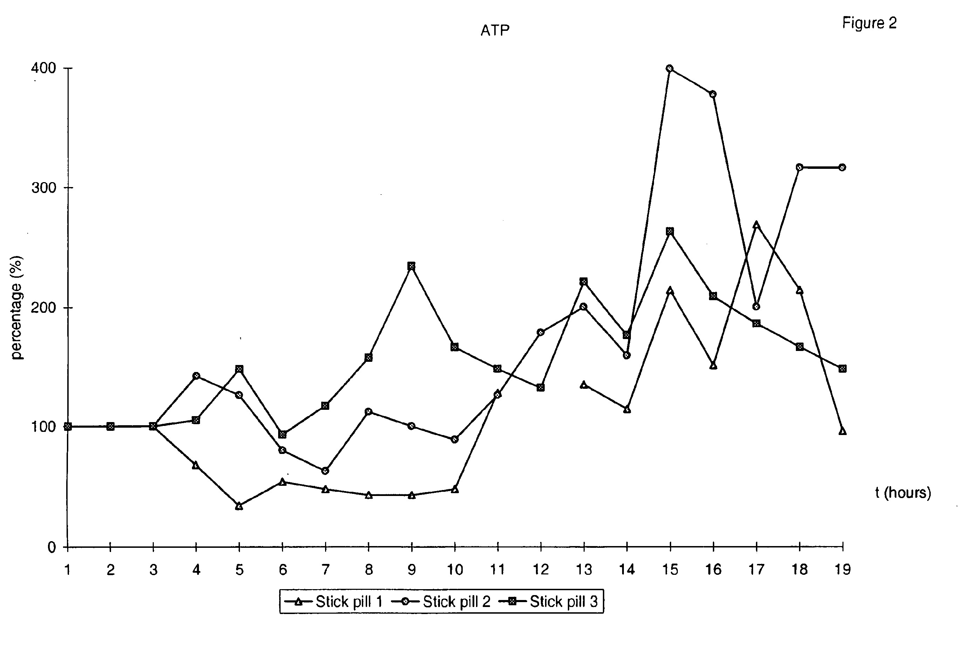

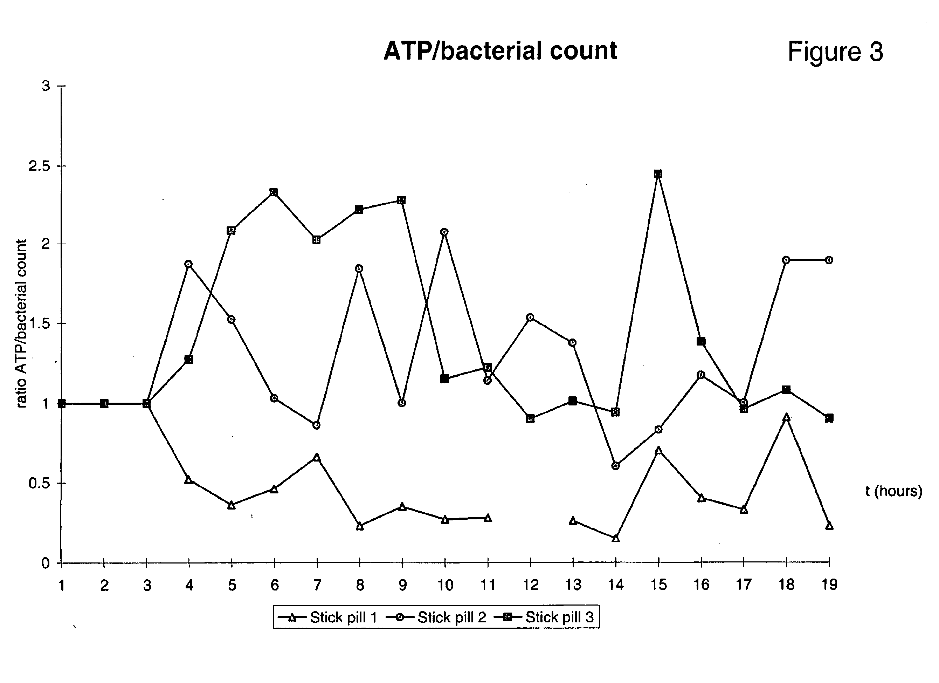

The saliva samples were taken off at different places of the mouth for each patient such as under the tongue, close to the crevicular canals). Each sample was divided in two parts: one to analyse the total microbial count on a Petri dish, the other one to analyse the ATP content of the micro-organisms. The results are shown in Figures 1 and 2. As could be observed in Figure 1, the microbial count is more important for the Stick 1 than for the Stick 2 and Stick 3. It could be reasoned that the presence of the growth factors can facilitate the growth of the bacteria in the saliva. When the ATP energy of these micro-organisms contained in the samples was analyzed, it was difficult to observe important differences between the three groups (Figure 2). On the other hand, when the ratio ATP/microbial count was analyzed, the results show an important difference characterised by much lower values for the Stick 1 compared to the Stick 2 and Stick 3 (Figure 3).

Regarding the values of the microbial count (Fig 1), it could be expected to find lower values for Stick 1 and Stick 2 containing the active ingredients. Observing the ratio ATP/microbial count (Fig 3), it can be concluded that if the microbial count is higher for the Stick 1, it is due to the detachment of the bacterial biofilms linked to the mucosa cells which allows these isolated bacteria to be attacked and inhibited by the enzyme group. In the case of Stick 2, the action of the enzyme group is performed only on the isolated bacteria contained in the saliva but it is evident that regarding the microbial count and ATP energy values for this experiment, the enzyme group alone has no effect on the bacteria colonized in biofilms. This corresponds to the conclusions made by different researchers, which observe an action of the enzyme group in some experiments and not in other experiments because they never compared the difference between the isolated bacteria and the bacteria colonised in biofilms.

Eventually for Stick 3, as a control, it can be concluded that few bacteria can be eliminated by the saliva and certainly not the ones which are colonized under biofilm forms.

### Example VI

The same experiment as described in Example V was performed using a toothpaste containing the enzyme group and the growth factor group (Toothpaste 1) which was compared to a toothpaste containing only the enzyme group (Toothpaste 2) and a toothpaste containing no enzyme group and no growth factor group (Toothpaste 3).

### Toothpaste with active ingredients Enzymes and Growth Factors : Toothpaste 1

| Active ingredients | Amounts per 100 g of paste |
|---|---|
| Lactoperoxidase | 10,000 ABTS units |
| Glucose Oxidase | 10,000 ABTS units |
| Substrate (thiocyanate) | 50 mg |
| Lactoferrin | 100 mg |
| Lysozyme | 100 mg |
| Immunoglobulins | 300 mg |
| Derived Platelet growth factor | 100 ng |
| Transforming growth factor | 120 ng |
| Fibroblast growth factor | 50 ng |
| Epidermal growth factor | 1 ng |
| Angiogenin | 1 ng |

### Non active ingredients

Sorbitol, glycerine, silica, carboxymethylcellulose, sodium benzoate, xylitol, flavour, titane dioxide

Toothpaste with only the Enzymes as active ingredients : Toothpaste 2

| Active ingredients | Amounts per 100 g of paste |
|---|---|
| Lactoperoxidase | 10,000 ABTS units |
| Glucose Oxidase | 10,000 ABTS units |
| Substrate (thiocyanate) | 50 mg |
| Lactoferrin | 100 mg |
| Lysozyme | 100 mg |
| Immunoglobulins | 300 mg |

### Non active ingredients

### Sorbitol, glycerine, silica, carboxymethylcellulose, sodium benzoate, xylitol, flavour, titane dioxide

Toothpaste where the active ingredients are replaced by sorbitol : Toothpaste 3 (control)

### Non active ingredients

### Sorbitol, glycerine, silica, carboxymethylcellulose, sodium benzoate, xylitol, flavour, titane dioxide

The patients suffering from a dry mouth because they produced less than 0,2 ml per minute of saliva brushed their teeth for at least two minutes with the toothpastes 1 to 3. The patients rinsed their mouth with only 5 ml water and a sample is taken off. 24 patients were used in each group. The saliva samples were taken off at different places of the mouth for each patient such as under the tongue, close to the crevicular canals. Each sample was divided in two parts: one to analyse the total microbial count on a Petri dish, the other one to analyse the ATP content of the micro-organisms.

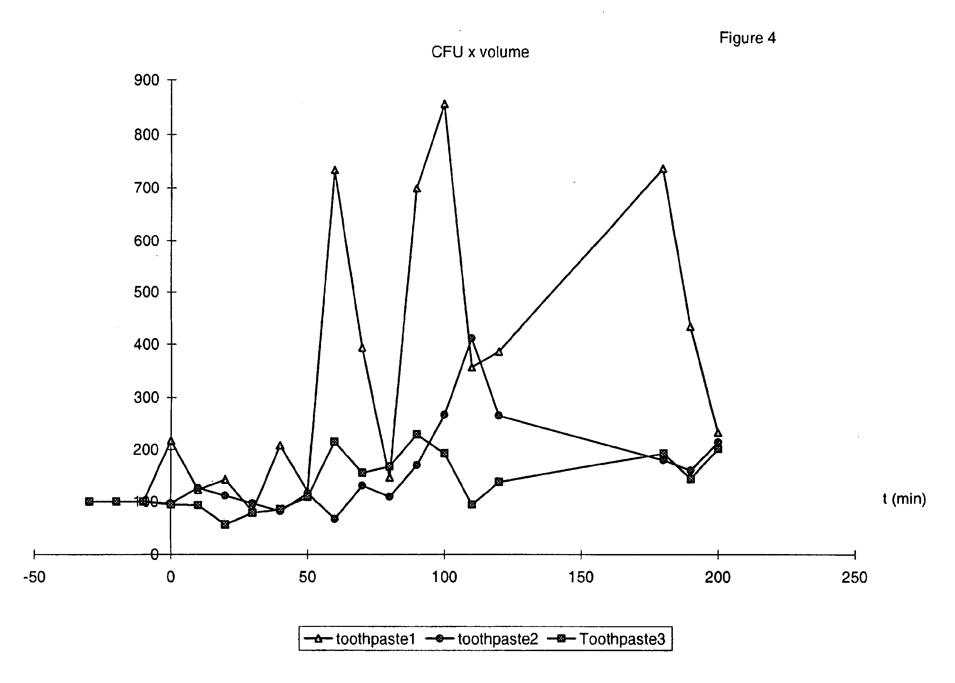

As can be observed, the microbial count is more important for the Toothpaste 1 than for the Toothpaste 2 and Toothpaste 3 (Figure 4). It could be reasoned that the presence of the growth factors can facilitate the growth of the bacteria in the saliva.

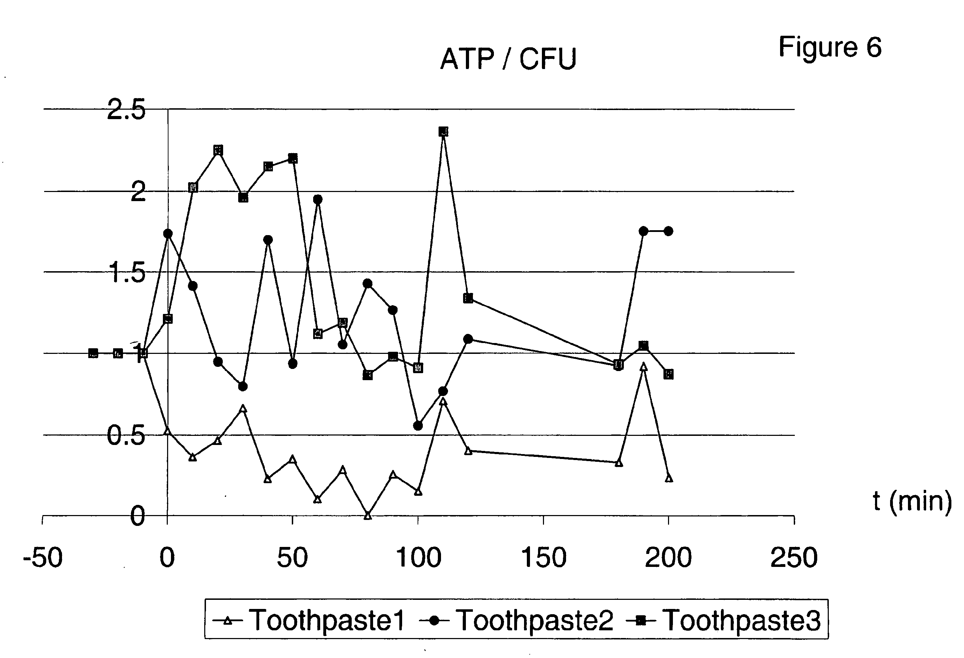

When the ATP energy of these micro-organisms contained in the samples was analyzed, it was difficult to observe important differences between the three groups.
On the other hand, when the ratio ATP/microbial count is analyzed, the results show an important difference characterised by a much lower values for the Toothpaste 1 compared to the Toothpaste 2 and Toothpaste 3 (Figure 6).

Regarding the values of the microbial count (Fig 4), it could be expected to find lower values for the Toothpaste 1 and Toothpaste 2 containing the active ingredients. Observing the ratio ATP/microbial count (Fig 6), it can be concluded that if the microbial count is higher for the Toothpaste 1, it is due to the detachment of the bacterial biofilms linked to the mucosa cells and allow to these isolated bacteria to be attacked and inhibited by the enzyme group. In the case of the Toothpaste 2, the action of the enzyme group is performed only on the isolated bacteria contained in the saliva but it is evident that regarding the microbial count and ATP energy values for this experiment, the enzyme group alone has no effect on the bacteria colonised in biofilms. This corresponds to the conclusions made by different researchers, which observe an action of the enzyme group in some experiments and not in other experiments because they never compared the difference between the isolated bacteria and the bacteria colonised in biofilms.

Eventually for the Toothpaste 3, as control, we can conclude that few bacteria can be eliminated by the saliva and certainly not the ones which are colonised under biofilm forms.

### Example VII

| Sterilant solution | concentrated powder |
|---|---|
| Lactoferrin peptides | 100 mgr |
| Epidermal factor | 10 µgr |
| Dialkyldimethylammonium chloride | 5 gr |

### Example VIII

| Sterilant solution | concentrated tablet |
|---|---|
| Lactoferrin peptides | 250 µgr |
| Bovine whey colostrum | 10 mgr |
| Sodium bicarbonate | 1000 mgr |
| Polyvinylpyrrolidone | 50 mgr |
| Tartric acid | 1000 mgr |

The bovine whey colostrum results of the action of skimmed colostrum and calf rennet. This action precipates some proteins from the colostrum. The supernatant is the whey.

### Example IX

| Sterilant solution | concentrated tablet |
|---|---|
| Lactoferrin | 1 mgr |
| Bovine whey colostrum | 10 mgr |
| Propylene glycol | 989 mgr |

Lactoferrin and bovine whey colostrum is dissolved in propylene glycol to give 1 gr of concentrated sterilant solution. 1 gr of concentrated sterilant solution is dispensed from a measured dose bottle, measured dose pump pack, polymer or glass phial to be diluted with 90 ml of water to give a sterilant solution.

### Example X

| Toothpaste tablets | |
|---|---|
| Flavour | 0.06 mgr |
| Sodium fluoride | 0.15 mgr |
| Micro-silicium | 4.5 mgr |
| Lactoferrin peptides | 5 mgr |
| Bovine colostrum | 15 mgr |
| Amorphous silicium | 22.5 mgr |
| Mg stearate | 52.5 mgr |
| Xylitol | 600 mgr |
| Sorbitol DC 60/W | 739.515 mgr |

Considering that bovine colostrum contains the growth factors, the activity of this toothpaste is due to a synergistic effect between lactoferrin peptides and the bovine colostrum.

### Example XI

| Aqueous cream for cosmetic use | |
|---|---|
| Lactoferrin peptides | 10 mgr |
| Bovine whey colostrum | 100 mgr |
| Emulsifying wax | 9 gr |
| Liquid paraffin | 6 gr |
| White soft paraffin | 10 gr |
| Purified water | to 100 gr |

### Example XII

| Enteric-coated tablet | |
|---|---|
| Hydroxypropyl methylcellulose phthalate | 6 mgr |
| Bovine whey colostrum | 100 mgr |
| Lactoferrin peptides | 1 mgr |
| Cetyl alcohol | 0.5 mgr |

The enteric coating solution is for example applied on the whey colostrum core by fluidized bed coating.

## Claims

1. Use of a composition comprising a combination of at least one compound chosen from:
the group of peroxidase, lactoferrin, lactoferrin peptides, lysozyme and immunoglobulins and a growth factor for the preparation of a medicament for prophylaxis and therapy of infectious diseases caused by microorganisms present in biofilms adherent to cell surfaces.

2. Use according to claim 1, further **characterized in that** said growth factor is chosen from platelet derived growth factor, fibroblast growth factor, transforming growth factor, angiogenin and epidermal growth factor.

3. Use according to claim 1, further **characterized in that** at least a peroxide is combined with a growth factor according to claim 2.

4. Use according to claim 1, further **characterized in that** at least a lactoferrin is combined with a growth factor according to claim 2.

5. Use according to claim 1, further **characterized in that** at least lysozyme is combined with a growth factor according to claim 2.

6. Use according to claim 1, further **characterized in that** at least immunoglobulin is combined with a growth factor according to claim 2.

7. Use according to claim 1, further **characterized in that** a lactoferrin peptide is combined with a growth factor according to claim 2.

8. Use according to any of claims 3 to 7, further **characterized in that** said growth factor is platelet derived growth factor.

9. Use according to any of claims 3 to 7, further **characterized in that** said growth factor is fibroblast growth factor.

10. Use according to any of claims 3 to 7, further **characterized in that** said growth factor is transforming growth factor.

11. Use according to any of claims 3 to 7, further **characterized in that** said growth factor is epidermal growth factor.

12. Use according to any of claims 3 to 7, further **characterized in that** said growth factor is angiogenin.

13. Use according to any of claims 1 to 12, wherein the composition comprises whey colostrum and/or colostrum as a source for a growth factor.

14. Use according to any of claims 1 to 13, further **characterized in that** said medicament is in the form of a gel, a stick pill, a rinsing liquid, a toothpaste, a tablet, a topical medicament, an oral dentifrice, an injectable composition, an oral tablet, a lozenge or a soft gelatin capsule.

## Patentansprüche

1. Verwendung einer Zusammensetzung, umfassend eine Kombination aus zumindest einer Verbindung, ausgewählt aus der Gruppe aus Peroxidase, Lactoferrin, Lactoferrinpeptiden, Lysozym und Immunoglobulinen, und einem Wachstumsfaktor zur Herstellung eines Medikaments zur Prophylaxe und Therapie von Infektionskrankheiten, die durch Mikroorganismen hervorgerufen werden, die in an Zelloberflächen haftenden Biofilmen vorhanden sind.

2. Verwendung nach Anspruch 1, ferner **dadurch gekennzeichnet, dass** der Wachstumsfaktor aus einem von Thrombozyten abgeleitetem Wachstumsfaktor, einem Fibroblasten-Wachstumsfaktor, einem transformierendem Wachstumsfaktor, Angiogenin und einem epidermalem Wachstumsfaktor ausgewählt ist.

3. Verwendung nach Anspruch 1, ferner **dadurch gekennzeichnet, dass** zumindest ein Peroxid mit einem Wachstumsfaktor nach Anspruch 2 kombiniert ist.

4. Verwendung nach Anspruch 1, ferner **dadurch gekennzeichnet, dass** zumindest ein Lactoferrin mit einem Wachstumsfaktor nach Anspruch 2 kombiniert ist.

5. Verwendung nach Anspruch 1, ferner **dadurch gekennzeichnet, dass** zumindest Lysozym mit einem Wachstumsfaktor nach Anspruch 2 kombiniert ist.

6. Verwendung nach Anspruch 1, ferner **dadurch gekennzeichnet, dass** zumindest Immunglobulin mit einem Wachstumsfaktor nach Anspruch 2 kombiniert ist.

7. Verwendung nach Anspruch 1, ferner **dadurch gekennzeichnet, dass** zumindest ein Lactoferrinpeptid mit einem Wachstumsfaktor nach Anspruch 2 kombiniert ist.

8. Verwendung nach einem der Ansprüche 3 bis 7, ferner - **dadurch gekennzeichnet, dass** es sich beim Wachstumsfaktor um einen von Thrombozyten abgeleiteten Wachstumsfaktor handelt.

9. Verwendung nach einem der Ansprüche 3 bis 7, ferner **dadurch gekennzeichnet, dass** es sich beim Wachstumsfaktor um einen Fibroblasten-Wachstumsfaktor handelt.

10. Verwendung nach einem der Ansprüche 3 bis 7, ferner **dadurch gekennzeichnet, dass** es sich beim Wachstumsfaktor um einen transformierenden Wachstumsfaktor handelt.

11. Verwendung nach einem der Ansprüche 3 bis 7, ferner **dadurch gekennzeichnet, dass** es sich beim Wachstumsfaktor um einen epidermalen wachstumsfaktor handelt.

12. Verwendung nach einem der Ansprüche 3 bis 7, ferner **dadurch gekennzeichnet, dass** es sich beim Wachstumsfaktor um Angiogenin handelt.

13. Verwendung nach einem der Ansprüche 1 bis 12, wobei die Zusammensetzung Molken-Colostrum und/oder Colostrum als Quelle für einen Wachstumsfaktor umfasst.

14. Verwendung nach einem der Ansprüche 1 bis 13, ferner **dadurch gekennzeichnet, dass** das Medikament in Form eines Gels, einer "Stick"-Pille, einer Spülflüssigkeit, einer Zahnpaste, einer Tablette, eines topischen Medikaments, eines oralen Zahnpflegemittels, einer injizierbaren Zusammensetzung, einer oralen Tablette, einer Pastille oder einer Weichgelatinekapsel vorliegt.

## Revendications

1. Utilisation d'une composition comprenant une combinaison d'au moins un composé choisi dans le groupe constitué par la peroxydase, la lactoferrine, les peptides de lactoferrine, le lysozyme et les immunoglobulines, et d'un facteur de croissance pour la préparation d'un médicament destiné à la prophylaxie et au traitement de maladies infectieuses provoquées par des microorganismes présents dans des biofilms adhérant à des surfaces cellulaires.

2. Utilisation selon la revendication 1, **caractérisée en outre en ce que** ledit facteur de croissance est choisi parmi le facteur de croissance dérivé des plaquettes, le facteur de croissance fibroblastique, le facteur de croissance transformant, l'angiogénine et le facteur de croissance épidermique.

3. Utilisation selon la revendication 1, **caractérisée en outre en ce qu'**au moins un peroxyde est combiné avec un facteur de croissance selon la revendication 2.

4. Utilisation selon la revendication 1, **caractérisée en outre en ce qu'**au moins une lactoferrine est combinée avec un facteur de croissance selon la revendication 2.

5. Utilisation selon la revendication 1, **caractérisée en outre en ce qu'**au moins le lysozyme est combiné avec un facteur de croissance selon la revendication 2.

6. Utilisation selon la revendication 1, **caractérisée en outre en ce qu'**au moins l'immunoglobuline est combinée avec un facteur de croissance selon la revendication 2.

7. Utilisation selon la revendication 1, **caractérisée en outre en ce qu'**un peptide de lactoferrine est combiné avec un facteur de croissance selon la revendication 2.

8. Utilisation selon l'une quelconque des revendications 3 à 7, **caractérisée en outre en ce que** ledit facteur de croissance est le facteur de croissance dérivé des plaquettes.

9. Utilisation selon l'une quelconque des revendications 3 à 7, **caractérisée en outre en ce que** ledit facteur de croissance est le facteur de croissance fibroblastique.

10. Utilisation selon l'une quelconque des revendications 3 à 7, **caractérisée en outre en ce que** ledit facteur de croissance est le facteur de croissance transformant.

11. Utilisation selon l'une quelconque des revendications 3 à 7, **caractérisée en outre en ce que** ledit facteur de croissance est le facteur de croissance épidermique.

12. Utilisation selon l'une quelconque des revendications 3 à 7, **caractérisée en outre en ce que** ledit facteur de croissance est l'angiogénine.

13. Utilisation selon l'une quelconque des revendications 1 à 12, dans laquelle la composition comprend du colostrum de lactosérum et/ou du colostrum en tant que source de facteur de croissance.

14. Utilisation selon l'une quelconque des revendications 1 à 13, **caractérisée en outre en ce que** ledit médicament est sous la forme d'un gel, d'une pilule adhésive, d'un liquide de rinçage, d'une pâte dentifrice, d'un comprimé, d'un médicament topique, d'un dentifrice à usage oral, d'une composition injectable, d'un comprimé à usage oral, d'une pastille ou d'une capsule de gélatine molle.
